(19) Europäisches Patentamt
European Patent Office
Office européen des brevets

(11) **EP 3 937 901 B1**

(12) **EUROPEAN PATENT SPECIFICATION**

(45) Date of publication and mention
of the grant of the patent:
**15.03.2023  Bulletin 2023/11**

(21) Application number: **20705088.1**

(22) Date of filing: **21.02.2020**

(51) International Patent Classification (IPC):
**A61K 9/00** (2006.01)        **C12N 9/52** (2006.01)
**C12P 19/14** (2006.01)

(52) Cooperative Patent Classification (CPC):
**A61K 9/0053; C12N 1/20; C12P 7/6463**

(86) International application number:
**PCT/EP2020/054579**

(87) International publication number:
**WO 2020/187526 (24.09.2020 Gazette 2020/39)**

(54) **METHOD FOR PRODUCING A COMPOSITION COMPRISING ARCHAEAL LIPIDS FROM A SULFOLOBUS CELL CULTURE**

VERFAHREN ZUR HERSTELLUNG EINER ZUSAMMENSETZUNG MIT ARCHÄISCHEN LIPIDEN AUS EINER SULFOLOBUS-ZELLKULTUR

PROCÉDÉ DE PRODUCTION D'UNE COMPOSITION COMPRENANT DES LIPIDES D'ARCHAEA PROVENANT D'UNE CULTURE DE CELLULES DE SULFOLOBUS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **15.03.2019  EP 19163020**

(43) Date of publication of application:
**19.01.2022  Bulletin 2022/03**

(73) Proprietor: **TECHNISCHE UNIVERSITÄT WIEN 1040 Wien (AT)**

(72) Inventors:
• **QUEHENBERGER, Julian**
  **1060 Wien (AT)**
• **WURM, David**
  **1060 Wien (AT)**
• **SPADIUT, Oliver**
  **1060 Wien (AT)**

(74) Representative: **SONN Patentanwälte OG Riemergasse 14 1010 Wien (AT)**

(56) References cited:
**WO-A1-2007/112567     DE-A1-102012 216 378 US-A- 5 098 588**

• **PATEL G B ET AL: "ARCHAEOBACTERIAL ETHER LIPID LIPOSOMES (ARCHAEOSOMES) AS NOVEL VACCINE AND DRUG DELIVERY SYSTEMS", CRC CRITICAL REVIEWS IN BIOTECHNOLOGY, CRC PRESS, BOCA RATON, FL, US, vol. 19, no. 4, 1 January 1999 (1999-01-01), pages 317-357, XP000993262, ISSN: 0738-8551, DOI: 10.1080/0738-859991229170 cited in the application**

**Description**

The present invention relates to the production of compositions comprising archaeal lipids and compositions comprising archaeal lipids.

Liposomes are spherical lipid bilayer vesicles (lipid esters) surrounding an aqueous space. They are important carriers for the administration of drugs, vaccines, genes, proteins, small molecules, antibiotics and nutrients. Liposomes are made of phospholipids, mainly phosphatidylcholine, and cholesterol, but might also include other lipids, like phosphatidylethanolamine. Liposomes are generated by a large number of different methods (reviewed e.g. by van Hoogevest, Eur. J. Pharm. Sci. 108 (2017), 1-12; Szoka et al., Ann. Rev. Biophys. Bioeng. 9 (1980), 467-508), for example by dispersing phospholipids in aqueous medium, e.g. using mechanical treatment (e.g. in a homogenizer, preferably by high pressure homogenization) or sonication. They vary between 0.02 to 10 um in diameter. Their size and composition significantly affect their properties.

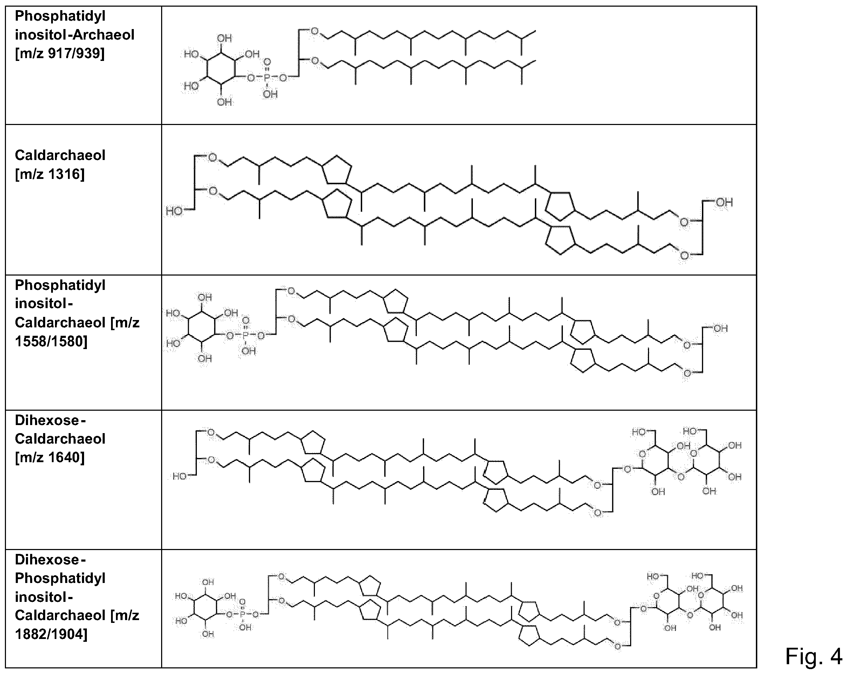

Archaeosomes (i.e. lipid vesicles which are composed partly or completely of archaeal lipids) represent a special class of liposomes originating from membrane lipids isolated from Archaea. Archaeosomes are made of lipid ethers, namely diether structures (archaeols) or tetraether structures (caldarchaeols) (e.g. Kaur et al., Drug delivery 23 (2016), 2497; Patel et al., Critical Reviews in Biotechnology 19 (1999), 317; Fig. 4). Diether structures are composed of a glycerol moiety carrying two phytanyl chains (20-40 carbons in length) on the sn-2,3 positions. Tetraether structures carry two di-phytanyl chains linked to two glycerol residues in either an antiparallel manner (caldarchaeol) or a parallel manner (isocaldarchaeol). Furthermore, one or several cyclopentane rings may occur. In general, archaeosomes are made of a large range of heterogeneous lipid structures, which significantly affects their characteristics. Depending on the composition (amount of archaeols vs. caldarchaeols), the lipid layer is a mono- or a bi-layer or a mixture thereof. The total lipids of Archaea, obtained by solvent extraction, account for about 5% of the cell dry weight. As shown in several studies, the type and structure of archaeal lipids depend on the organism they are extracted from and their respective living environments (e.g. temperature) as well as growth phases (Jensen et al., Life 5 (2015), 1539). For example, an increase in growth temperature for *M. jannaschii* from 50 to 65°C resulted in the percent core lipid distribution of archaeol, caldarchaeol and macrocyclic archaeol to shift from 60, 21, and 19% to 15, 42, and 43%, respectively (Sprott et al., J. Bact. 173 (1991), 3907). Also for the Archaea *Sulfolobus* it is speculated that the membrane fluidity is controlled by the number of cyclopentane rings within the phytanyl chains of the caldarchaeol lipids, which increases in response to higher growth temperatures (Jensen et al., 2015).

US 5,098,588 A discloses the use of archaebacterial lipids as a new class of lubricants for metal surfaces. Neither a cultivation process for the archaebacteria is disclosed in this document not are compositions with specific quantitative amounts of different archaeal lipids provided. WO 2007/112567 A1 discloses a polar synthetic lipid comprising at least one carbohydrate or anionic group linked by covalent bonding to at least one free hydroxyl group of an archaeal core lipid, wherein *Thermoplasma* is used as a source of caldarchaeol lipids. Using those lipids from *Thermoplasma* is disclosed in this document to be specifically preferred compared to using *Sulfolobus* as a source, to avoid mixtures of caldarchaeols and nonitol-caldarchaeols found in *Sulfolobus*. DE 10 2012 216 378 A1 describes an immobilisation matrix comprising a tetraether lipid layer, on which ligands (capture molecules) that can bond to a target can be covalently immobilised and biosensor chips, for chip-based analysis methods, which comprise such matrix.

Currently, archaeosomes are receiving increased attention as they have proven to be highly stable against heat, pH, bile salts, lipases and oxidation, making them more attractive than conventional liposomes. In several *in vitro* studies, it was demonstrated that archaeosomes can be used as versatile nano-carrier systems for different cargo with a huge prospect to fight various diseases. However, archaeosomes have not seen their breakthrough in application yet due to the following limitations:

   1) They are produced in a complex environment (complex cultivation media and uncontrolled shake flask cultivations) and are thus very heterogeneous,
   2) they cannot be produced under defined conditions contradicting regulatory guidelines (Quality by Design),
   3) the lipid composition of the Archaea cannot be controlled,
   4) they cannot be produced cost-efficiently in high amounts.

It is therefore an object of the present invention to reduce the heterogeneity of archaeosomes and enable generation of tailor-made archaeal lipids by process parameters under controlled cultivation conditions for Archaea. More specifically, the object of the present invention is to provide an archaeosome composition which comprises many cyclopentane ring lipids but at the same time also phosphatidyl archaeol. It is a further object to provide compositions of archaeal lipids which are suitable for specific delivery route and/or administration, e.g. for oral retard delivery and/or for oral acute delivery. It is a further object to provide archaeal liposome compositions which have an appropriate stability (defined e.g. as the ability to stably wrap the drug of interest and enable stomach passage or skin passage or passage of mucosa) of the archaeosomes and/or sufficient functionality (defined e.g. as ratio between caldarchaeols and archaeols). Another aim of the present invention is the preparation of a lipid mixture with the highest possible number of rings in the phytanyl chains of the caldarchaeol species while maintaining the adjustability of the ratio caldarchaeol: Archaeol in the liposomes derived from Archaea (within the physiological limits).

Accordingly, the present invention provides cultivation methods for *Sulfolobus* on a defined medium und controlled process conditions for a certain, more homogeneous lipid composition to generate tailor-made archaeosomes with reduced heterogeneity as vesicles for the biopharmaceutical industry.

More specifically, the present invention provides a method for producing a composition comprising archaeal lipids from a *Sulfolobus* cell culture, comprising

(1) growing a *Sulfolobus* cell culture at a temperature of 75 to 85°C and at a growth rate of 0.025 to 0.035 per hour and extracting the archaeal lipids from the archaeal culture so as to obtain a composition for use for oral retard therapy, or

(2) growing a *Sulfolobus* cell culture at a temperature of 75 to 85°C and at a growth rate of 0.005 to 0.015 per hour and extracting the archaeal lipids from the archaeal culture so as to obtain a composition for use for oral acute therapy.

Archaeosomes have advantageous properties which have made them as promising candidates for future pharmaceutical in the prior art. However, up to now, the major obstacles from making them practically relevant candidates were mainly the lack of controllability of the production processes and their high variability concerning their lipid ether composition. The present invention now enables such industrial approach making archaeosomes realistic candidates for drug cargo intended to be delivered to human patients:

For human therapeutic applications it is generally required that formulated drugs have a minimum shelf-life of 1-2 years. This implies physical stability (aggregation, loss of cargo) and chemical stability during storage (oxidation, hydrolysis). In recent years, the use of liposomes in drug/vaccine delivery has been extended to oral delivery system. Conventional liposomes cannot be used for this purpose due to their poor stability in the harsh environment of the gastrointestinal (GI) tract (pH as low as 1 in the stomach and attack by lipases and bile salts.) The main advantage of archaeosomes compared to conventional liposomes is their high stability. Different stability aspects have been tested, demonstrating the benefits of archaeosomes over liposomes:

- Storage stability: Archaeosomes contain saturated phytanyl chains and are thus not oxidized by air. Consequently, they can be stored in chloroform/methanol at room temperature without any alteration for years.
- Thermal stability: Comparative thermal stability of archaeosomes made from e.g. *Sulfolobus acidocaldarius* with conventional liposomes showed that unlike the conventional liposomes that became permeable at 35-40°C and lost their cargo, archaeosomes did not get leaky at much higher temperatures. Cyclic structures in the archaeal lipids, which are deposited as a thermo-adaptive response, confer tight membrane packing and reduced membrane fluidity. This high thermal stability allows sterilization, which is a crucial requirement for *in vivo* applications of lipid vesicles, by autoclaving. In contrast, heat sterilization of conventional liposomes causes aggregation, hydrolysis and leakage, making the preparation of sterile liposomes a cumbersome endeavor.
- pH stability: *In vitro* assays revealed that archaeosomes are more stable at acidic pH than conventional liposomes: after storage at pH 3.0 for 21 days, liposomes lost up to 60% of their cargo, whereas archaeosomes only lost 30%.
- Stability against lipases: Lipases in the GI tract cause severe hydrolysis of liposomes, resulting in a rapid release of the encapsulated cargo. Previous studies showed that conventional liposomes lost up to 100% of cargo when exposed to phospholipase A2 for 4 hours, whereas archaeosomes lost less than 20%.
- Stability in the presence of bile salts: It was shown that archaeosomes retain all their cargo during a 90 min incubation at 37°C in simulated human bile, which is longer than the average residence time of lipid vesicles in the stomach.
- Stability against the combined effect of lipases and bile salts: In the GI tract, the lipid vesicles meet both lipases and bile salts affecting their stability. Conventional liposomes lose 100% of their cargo when incubated in the presence of pancreatic lipase in simulated human bile already after 30 min, whereas lipases and bile salts do not exhibit a synergistic effect on archaeosomes.

Summarizing, archaeosomes already had the potential to out-compete currently used liposomes regarding their stability (more stable over time and more resistant against heat, pH, lipases, bile acid, autoclavable). With the present invention, a reliable method is provided to enable suitable and reproducible compositions for specific delivery of drugs via archaeosomes. The present invention therefore enables production of archaeosomes in sufficient quantities at sufficient quality.

Archaeosomes have been recognized as tremendously useful carriers for the delivery of a variety of cargo. The drug-carrier application, which currently is most promising, is the use of archaeosomes as microcapsules for oral delivery of therapeutic proteins or peptides (like insulin). To date, they are also regarded as highly interesting adjuvants in vaccines since the uptake of archaeosomes in phagocytic cells is more than 50-fold greater than of conventional liposomes. Studies in mice show that systemic administration of several test antigens entrapped in archaeosomes gave humoral immune responses that were comparable to those obtained with the potent, but toxic Freund's adjuvant. All *in vitro* and *in vivo* studies performed to date indicate that archaeosomes are safe and do not invoke any noticeable toxicity. In general, requirements for lipid carriers in medical applications are:

- Safety, stability, bioavailability, cost effectiveness
- No toxicological effects on the human body
- Induction of nonpathological inflammatory responses
- Promotion of cross-talk between innate and acquired immune system
- Augmentation of humoral antibody response and induction of T-cell mediated response

Archaeosomes fulfill all of these requirements and thus have been successfully tested as drug delivery systems for cancer vaccines, immunoadjuvants against Chagas disease, novel gene delivery systems, carriers for oral delivery of proteins and peptides, novel antigen delivery systems and others.

A broad range of potential applications of archaeosomes with medical relevance have been shown *in vitro* and in the animal model. However, due to insufficient homogeneity of the lipid composition clinical applications have not been realized yet.

Archaeosomes have been successfully prepared from different archaeal species. Usually, total archaeal lipids are extracted from frozen biomass by organic solvent extraction using chloroform/methanol/water. Then, the polar and the neutral lipids are separated by precipitation using acetone. The resulting lipid extracts can either be used directly for the preparation of archaeosomes as complex mixtures of different lipid classes or can be further purified by chromatography to isolate pure polar lipids of a particular class. Starting from these special, natural archaeal lipids, it is possible to prepare formulations of archaeosomes and encapsulate or associate hydrophilic or hydrophobic compounds using available methods developed for the preparation of conventional liposomes, like the mechanical dispersion method, the lipid hydration method, membrane extrusion or sonication. Methods for preparing lipids from Archaea or for cultivating Archaea are disclosed e.g. in US 2017/0152533 A1, US 6,316,260 B1, EP 1 999 137 B1, EP 0 883 624 B1, EP 2 109 459 B1, Siliakus et al. (Extremophiles 21 (2017), 651-670), Jain et al. (Frontiers in Microbiol. 5 (2014), article 641)).

Even though Archaea are known to be a useful source of these special lipids, they have not extensively been used for that purpose yet since currently the composition of the liposome cannot be controlled and is very heterogenous. Most Archaea are cultivated on complex medium in shake flasks under uncontrolled conditions which leads to batch-to-batch variations in terms of lipid class composition, lipid core structure as well as branching. Furthermore, the composition and the properties of the lipidome was up to now not controllable. However, this is essential for reproducible high-quality archaeosome formation with reduced heterogeneity and specific properties as demanded for pharmaceutical applications. It was observed that the lipid composition of *S. islandicus* and *S. tokodaii* was not constant in different processes, especially when the temperature was changed or samples were taken in different growth phases (Jensen et al., Life 5 (2015), 1539). By cultivating Archaea on a defined medium in the controlled environment of a bioreactor the present invention provides an efficient control of the quality (heterogeneity and composition) of the lipid ethers of the Archaea by changing critical process parameters (e.g. temperature, growth rate, etc.), which impedes the preparation of well-defined archaeosomes.

Archaeosomes can be used for similar tasks as liposomes (administration of drugs to patients), but show much better properties than liposomes (more stable over time and more resistant against heat, pH, bile acid, autoclavable) and are expected to be a viable alternative to conventional liposomes as they can be produced in sufficient quantities at sufficient quality.

The global liposome drug delivery market is valued at 2.4 billion USD in 2017 and expected to reach 5.2 billion USD by the end of 2025, growing at an annual growth rate of 10.1% during 2018-2025.

A recent review on the clinical use of liposomal formulations listed a total of 15 approved clinical products worldwide covering the areas anti-cancer, anti-fungal, vaccines, anti-inflammatory drugs and therapeutic genes.

For the use of archaeosomes as a carrier of pharmaceutical active ingredients, it is necessary to ensure a different release kinetics depending on the desired site of action and pharmaco-dynamics. This release kinetics can be influenced essentially by the stability of the archaeosomes.

For stability, in addition to the basic type of lipids, especially the ratio of short and long-chain lipids is crucial; as well as the possible presence of ring structures in the lipid chains which, by restricting chain mobility, cause more dense packing and reduction in membrane permeability. The combination of archaeol (Arc, a total of 40 C atoms in the phytanyl chains) and caldarchaeol (Cal, a total of 80 C atoms in the phytanyl chains) here represents a suitable lever. Both substances are natural membrane constituents of *Sulfolobus acidocaldarius* and other archaeal species. There are caldarchaeol variants with various modifications of the terminal glycerol residues (no modification, phosphatidylinositol (PI), dihexose (2Hex), and combinations thereof: Cal, PI-Cal, 2Hex-Cal, PI-Cal-2Hex). Furthermore, Cal variants with a different number of rings occur in the phytanyl chains (in the case of S. acidocaldarius, preferably 4, 5 or 6 rings).

Archaeol occurs mainly as PI-Arc.

For a number of reasons, a mixture of 2Hex-Cal and PI-Arc is a particularly interesting combination for the preparation of archaeosomes according to the present invention, especially for oral application:

- Caldarchaeols with different number of rings can be used.
- The mixture of short and long-chain lipids ensures influence on the stability of the formed archaeosomes.
- Both lipids have a sufficient number of functional groups in the head groups for efficient modification with linkers for use in targeted drug delivery (e.g. attachment of antibody fragments, aptamers, etc.).
- This modification can be carried out selectively on one of the two lipid classes, since the head groups differ in structure and chemical properties

As experimentally shown with the present invention, it is possible to influence the ratio Cal to Arc via the process parameters temperature and growth rate (maximum ratio Cal: Arc at optimal growth temperature and at highest growth rate and vice versa). The proportion of higher number of rings (6 rings vs. 5 or 4 rings), however, surprisingly depends exclusively on the temperature and does not correlate with the growth rate. The influence of temperature on the number of rings was observed in *Sulfolobus spp.* However, no literature is known about the influence of the growth rate.

Basically, the highest possible number of rings is necessary for the lowest possible membrane permeability and thus gastric resistance, which, as the present invention has shown, therefore requires a cultivation at the highest possible temperature. The associated loss of temperature as being a suitable means for establishing the Cal: Arc ratio, however, can be controlled according to the present invention via the growth rate. For the release of the drug in the intestine, the archaeosomes may not be too stable, because the drug otherwise cannot be delivered. Thus, the modifiability of the release kinetics of archaeosomal formulations according to the present invention is maintained and drugs produced on archaeosomes from the tailored lipid mixtures provided by the present invention, for example, after oral administration, have a different duration of action in the intestine.

The present invention is based on at least two significant results which have been obtained in the course of the present invention. First, the present invention provides a controlled production method which allows reproducible and defined compositions of archaeal lipids. Second, with this knowledge of applying and making use of the controlled production method for archaeal lipids (depending on specific and different cultivation conditions) according to the present invention, two specific kinds of archaeal compositions can be reproducibly provided also in upscaled formats by applying different cultivation conditions which are specifically useable (a) for oral retard therapy (i.e. for pharmaceutical compositions which show a retard effect or (b) for oral acute therapy (i.e. for pharmaceutical compositions which show immediate release to a patient when delivered orally) .

Bioreactor cultivation methods have been developed in the recent past together with new analyzation tools, computer-directed process-monitoring and software-guided process-controlling to achieve break-through technology in cultivation of microorganisms on completely different levels. This has specifically impacted the cultivation technology for microorganisms which require complex cultivation conditions, such as archaebacteria which e.g. require cultivation temperatures significantly above common cultivations temperatures, such as 30 or 37°C or even room temperature. Modern methods for observing process parameters which were not available ten or five years ago enable the methods according to the present invention as a necessary basis for the controllable and reproducible production process for the cultivation of archaeal cell cultures according to the present invention, especially for an effective controlling of the growth rate.

For example, Patel et al. (1999, above) report in table 1 the relative abundance (in %) of the main core lipids in representative archaebacteria. Due to the absence of appropriate analytical tools at that time, only core lipids were analysed semiquantitatively, i.e. as relative amounts. Apart from the low growth temperature (70°C; compared to an optimal growth temperature of at least 75°C), the lipids were obtained from late logarithmic growth phase, because a more specific growth phase simply was not controllable at that time but changed significantly during cultivation (Sprott et al., Can. J. Microbiol. 43 (1997), 467-476). It was not until the introduction of novel cultivation methods currently available that archaeal cell cultures were handleable to regulate cultivation parameters, such as a specific growth rate. Accordingly, the prior art was not even able to provide archaeal lipid compositions which were reproducibly provided in different - but always and reproducibly defined - qualitative and quantitative lipid entity compositions by different process parameters and/or different cultivation conditions. This is - for the first time for archaeal lipids derived from archaeal cell cultures, especially for *Sulfolobus* cell cultures - provided by the present invention.

With the present invention, the preparation of a lipid mixture with the highest possible number of rings in the phytanyl chains of the caldarchaeol species is possible while maintaining the adjustability of the ratio Cal: Arc (within the physiological limits). Due to their particular chemical suitability, 2Hex-Cal and PI-Arc are specifically preferred target species according to the present invention. With the present invention it is therefore possible to provide compositions with archaeosomes with a high number of rings due to increased growth temperature with simultaneous adjustment of the Cal: Arc ratio over the growth rate. Through controlled process conditions, the present invention makes it possible to obtain a lipid extract which can be either used directly or with minimum purification effort for the production of archaeosomes with high gastric juice resistance and tailor-made release kinetics for different pharmaceutical active ingredients.

With the present invention, two specific compositions comprising archaeal lipids from a *Sulfolobus* cell culture are provided, one being specifically suited for use for oral retard therapy, the other being specifically suited for use for oral acute therapy. The terms "oral retard therapy" and "oral acute therapy" are used according to the present invention in their usual meaning: Whereas a "retard" (or "time-release", "sustained release", "extended release") oral therapy shows a "retard effect" which enables a continuous release even after some time after delivery, e.g. 12 h after (oral) delivery, an "acute" (or "immediate release") oral therapy is immediately released after administration and has a release maximum immediately after delivery, e.g. within 3 h after oral administration (Pschyrembel, "retard effect": protracted release of an active substance due to galenic formulation; Andrade, J. Clin. Psychiatry 76 (2015), e995-e999; Perrie et al. "Controlling drug delivery" in Pharmaceutics: drug Delivery and Targeting" (2012), Pharmaceutical Press) .

The lipids produced for the production of oral archaeosomes with sustained-release effect are specifically suited for the needs of oral administration and therefore exhibit a high gastric juice resistance. This resistance is safeguarded by the presence of a high number of cyclopentane rings. According to the present invention, it was found that this can be achieved by applying high process temperatures, especially in the range of 75 to 85°C. For the retard effect necessary for sustained-release, a slow release of the active substance over a longer period is needed, which therefore requires temporal stability. According to the present invention, this can be achieved by the presence of less destabilizing PI-Arc. For obtaining this specific composition of lipids, the present invention provides the teaching that this requires a high growth rate, i.e. preferably a growth rate of 0.025 to 0.035 per hour, especially of 0.027 to 0.033 per hour. In a preferred embodiment of the present invention, the present method provides a composition for use for oral retard therapy which comprises a ratio of dihexose-caldarchaeol (2Hex-Cal) to phosphatidyl inositol-archaeol (PI-Arc) of 1:5 to 1:7, especially of 1:5.5 to 1:6.5.

The lipids produced for the production of oral archaeosomes for acute therapy are, on the one hand, also specifically suited for the needs of oral administration and therefore exhibit a high gastric juice resistance. This resistance is - as in the retard case - safeguarded by the presence of a high number of cyclopentane rings. According to the present invention, it was found that this can be achieved by applying high process temperatures, especially in the range of 75 to 85°C. On the other hand, a composition for acute therapy requires a rapid release of the drug in the intestine. Therefore, a lower stability of the composition is necessary. According to the present invention, this can be achieved by the presence of more destabilizing PI-Arc. For obtaining this specific composition of lipids, the present invention provides the teaching that this requires a low growth rate, i.e. preferably a growth rate of 0.005 to 0.015 per hour, especially of 0.007 to 0.013 per hour. In a preferred embodiment of the present invention, the present method provides a composition for use for oral acute therapy which comprises a ratio of dihexose-caldarchaeol (2Hex-Cal) to phosphatidyl inositol-archaeol (PI-Arc) of 1:2 to 1:4, especially of 1:2.5 to 1:3.5.

With these compositions obtainable by the present method, tailor-made archaeosomes can be provided wherein drugs to be delivered can be wrapped for administration, preferably for oral administration to human patients in need of an effective amount of this drug.

Culturing *Sulfolobus* cell cultures is well available to a person skilled in the art and is - in principle - an established technique for a considerable time (as early as Brock et al., Arch. Mikrobiol. 84 (1972), 54-68). A preferred *Sulfolobus* strain is *Sulfolobus acidocaldarius* cells; this is one of the established strains in the present field of technology. Depositaries for this *Sulfolobus* organism used may be, for example German strain collection for microorganisms (Deutsche Sammlung von Mikroorganismen und Zellkulturen, DSMZ), catalogue number DSM 639. An alternative source of supply is e.g. American Type Culture Collection (ATCC), catalogue number ATCC 33909. Other archaebacteria are also available e.g. at the DMSZ or the ATCC

After culturing the *Sulfolobus* cell cultures according to the defined culturing conditions, the *Sulfolobus* cell lipids are extracted. This can be done in the way as disclosed in the art. Preferably, the cells are homogenised after culturing, preferably via homogenization, especially high-pressure homogenization, or sonication, so that the lipids are extractable from the aqueous culture. A preferred method for purifying the lipids according to the present invention is the supercritical fluid extraction, especially the extraction with supercritical $CO_2$ (see e.g. Hanif et al., Int. J. Mol. Sci. 13 (2012), 3022-3037). Before extraction, it is preferred to mix the homogenised cells with a buffer, e.g. a buffer known and described in the art to be suitable for *Sulfolobus* cell lipids extraction, for example with ammonium acetate buffer, especially with an ammonium acetate buffer having 100 to 200 mM ammonium acetate.

According to a preferred embodiment of the present invention, the archaeal lipids are obtained by extraction of the aqueous phase comprising the cells and/or the homogenised cells into an organic phase, preferably by extraction into a chloroform/methanol organic phase, and phase separation of the organic phase from the aqueous phase. The organic phase then contains the lipids in the desired, "tailor-made" ratio. The archaeal lipids can then be obtained by evaporation of the organic phase, preferably by evaporation of the organic phase to dryness.

Preferably, the archaeal lipids obtained are combined with a pharmaceutically active drug, preferably in combination with a pharmaceutically acceptable carrier, so as to obtain a composition comprising archaeal lipids and the pharmaceutically active drug. This is preferably done after the purified composition comprising archaeal lipids (archaeosomes) are provided; it is, however, also possible to add the drug and/or further components already in the process of purification and co-purify it/them with the lipids.

According to another preferred embodiment of the present method, the archaeal lipids are obtained by supercritical fluid extraction, especially extraction with supercritical $CO_2$.

According to another aspect, the present invention also relates to a composition comprising archaeal lipids obtainable by a method according to the present invention. These "tailor-made" archaeosomal compositions are novel and of high utility for packaging drugs to be delivered to patients, especially for delivering drugs which are already known to be suitable for human therapeutic and prophylactic use.

The present invention also provides two specifically designed composition, one for the oral retard use and one for oral active use: Accordingly, preferred compositions according to the present invention comprise either a ratio of dihexose-caldarchaeol (2Hex-Cal) to phosphatidyl inositol-archaeol (PI-Arc) of 1:5 to 1:7 (retard oral use) or a ratio of dihexose-caldarchaeol (2Hex-Cal) to phosphatidyl inositol-archaeol (PI-Arc) of 1:2 to 1:4 (acute oral use). Both compositions, as defined by this specific 2Hex-Cal/PI-Arc ratio) have a high resistance against gastric juice which allows them an efficient passage of the stomach after oral administration. The retard oral composition further comprises less destabilising PI-Arc and has therefore a retarded release property in the intestine allowing a timely delayed release. The acute oral compositions are less stable in the intestine and the packaged drug can therefore immediately released from the liposomes, enabling a quick delivery for intestinal uptake of the drug. Unexpectedly, performing the culturing of the *Sulfolobus* cell culture at a growth rate of 0.020 per hour did neither result in a retard composition nor in an immediate release composition with the specific 2Hex-Cal/PI-Arc ratios as defined for the retard/acute compositions.

This was even more unexpected in view of Patel et al. (1999, above) wherein the data obtained for the lipid compositions e.g. for table 1 were obtained without controlling growth conditions (see e.g. Sprott et al., 1997, above).

The compositions according to the present invention are suitable to package any drug which is suitable for liposomal packaging. Accordingly, the compositions according to the present invention are specifically suited for further comprising a drug, preferably a drug for human medical use, especially a drug which is subject to an authorization to place the product on the market as a medicinal product issued in the EU or in the US. However, since there are virtually no physicochemical boundaries with respect to the drug to be combined in an archaeosomal preparation, any drug can be packaged in the lipid compositions according to the present invention. Accordingly, it is preferred to include drugs in the lipid compositions according to the present invention which are specified for human (or animal) use, be it because they are subject to a national or regional market authorization or be it because they are administered to humans based on another reason.

Preferred compositions according to the present invention comprise at least 5 % w/w, preferably at least 10 % w/w, PI-Arc and at least 50 % w/w 2Hex-Cal (% w/w as % w/w of total lipid).

According to a more general aspect, the present invention provides a method for producing a composition comprising archaeal lipids from an archaeal cell culture, comprising growing an archaeal cell culture at controlled conditions and extracting the archaeal lipids from the archaeal culture so as to obtain a composition comprising archaeal lipids. The present invention provides a controlled production method which allows reproducible and defined compositions of archaeal lipids. Preferably, the controlled conditions are selected from pH, growth rate, temperature, and process mode. The method according to the present invention is in principle applicable to all archaeal cells. Accordingly, the archaeal cell culture according to the present invention is a culture of *S. acidocaldarius, M. hungatei, M. voltae, M. concilii, M. smithii, M. espanolae, T. acidophilum, M. mazei, M. espanole, T. acidophilum, H. salinarum, M. smithii, M. stadtmanae, H. halobium, H. morrhuae, M. jannaschii, S. islandicus, S. solfataricus, S. shibatae, S. tokodaii, S. Metallicus, M. sedula, H. hispanica,* and *H.volcanii,* preferably *S. acidocaldarius.*

Preferred temperatures for growing the archaeal culture can be optimised using the teachings according to the present invention and are preferably in the range at a temperature of 55 to 90°C, preferably of 70 to 85°C, especially of 75 to 85°C.

Specifically preferred embodiments of the present invention comprise growing the archaeal culture at a growth rate of 0.025 to 0.035 per hour, especially of 0.027 to 0.033 per hour, or at a growth rate of 0.005 to 0.015 per hour, especially of 0.007 to 0.013 per hour.

Specifically preferred embodiments of the present invention comprise growing the archaeal culture at a pH of 2 to 4, preferably of 2.5 to 3.5.

The present invention can be applied by growing the archaeal culture at continuous mode and by growing the archaeal culture in batch mode, especially in fed-batch mode.

The method according to the present invention preferably comprises extracting the archaeal lipids by two phase extraction, organic extraction, especially Soxhlet, high pressure or high temperature extraction; by ionic liquids extraction, or by supercritical fluid extraction.

According to a preferred embodiment, the present method comprises extracting the archaeal lipids by a solvent selected from the group consisting of water, hexane, chloroform, acetone, diethyl ether, methanol, ethanol, ethyl acetate, dichloromethane, MTBE, $CO_2$, and mixtures thereof. The present invention is further illustrated by the following examples and the figures, yet without being limited thereto.

Fig. 1 shows the composition of the lipid pattern depending on growth temperature;
Fig. 2 shows the composition of the lipid pattern depending on growth rate;
Fig. 3 shows the composition of the lipid pattern depending on process mode;
Fig. 4 shows the structures main lipids of *Sulfolobus acidocaldarius*;
Fig. 5 shows the influence of growth temperature on the number of cyclopentane rings;
Fig. 6 shows the spectral data of biomass sample grown at 68°C;
Fig. 7 shows the spectral data of biomass sample grown at 75°C;
Fig. 8 shows the spectral data of biomass sample grown at 82°C;
Fig. 9 shows the spectral data of biomass sample grown with a growth rate of 0.010 h$^{-1}$;
Fig. 10 shows the spectral data of biomass sample grown with a growth rate of 0.020 h$^{-1}$;
Fig. 11 shows the spectral data of biomass sample grown with a growth rate of 0.034 h$^{-1}$;
Fig. 12 shows the spectral data of biomass sample grown in fed-batch mode;
Fig. 13 shows the spectral data of biomass sample grown in continuous mode.

Examples:

1. Generation of tailor-made archaeal lipids

The present invention describes a technology for the production of distinct lipid patterns in *Sulfolobus acidocaldarius* with a defined and tunable composition via variation of process parameters during the upstream process.

The present examples show that via variation of critical process parameters during the upstream, e.g. temperature, growth rate, process mode, the organism *Sulfolobus acidocaldarius* produced different lipid patterns (ratio of distinct lipid fractions). Thus, consequently different lipid compositions can be harvested after downstream processing. Since the lipid pattern can be influenced by a broad number of critical process parameters (temperature, growth rate, process mode, pressure, osmolarity, media composition, dissolved oxygen content, etc.) very specific tuning of the pattern is possible.

The following paragraphs describe showcases of the influence of (A) temperature, (B) growth rate and (C) process mode on the ratio of lipid fractions. Depending on the application of the lipid mix by the user/customer, a desired lipid pattern can be adjusted by carefully selecting the required process parameters.

Cultures were grown in a controlled bioreactor environment on a defined medium.

1.1 Influence of temperature

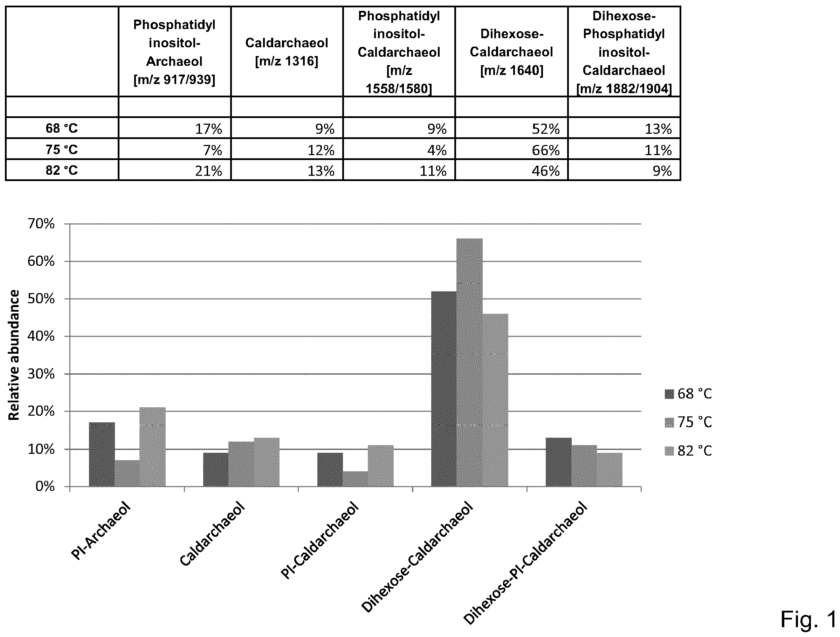

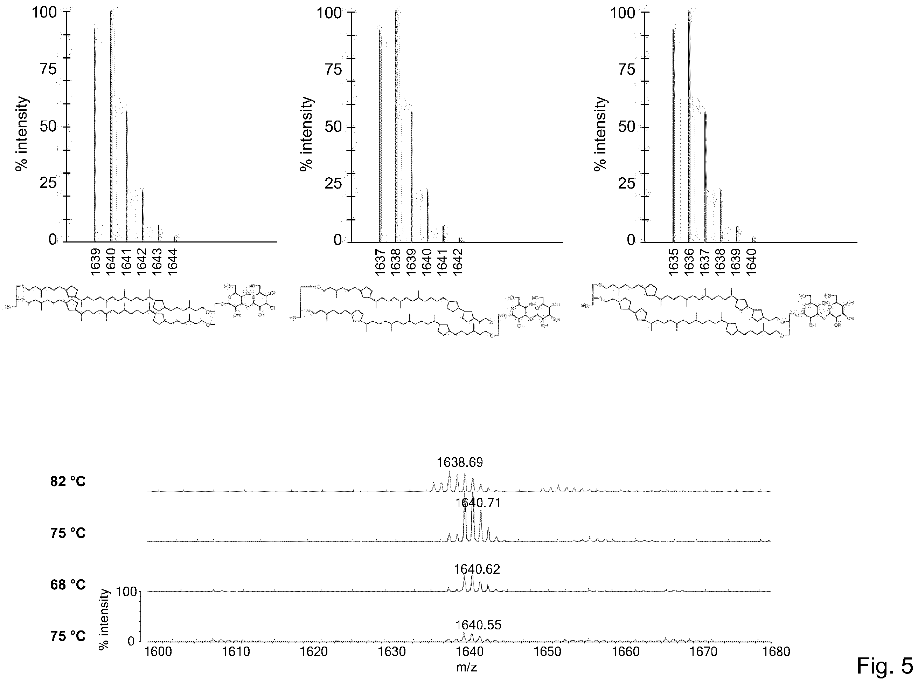

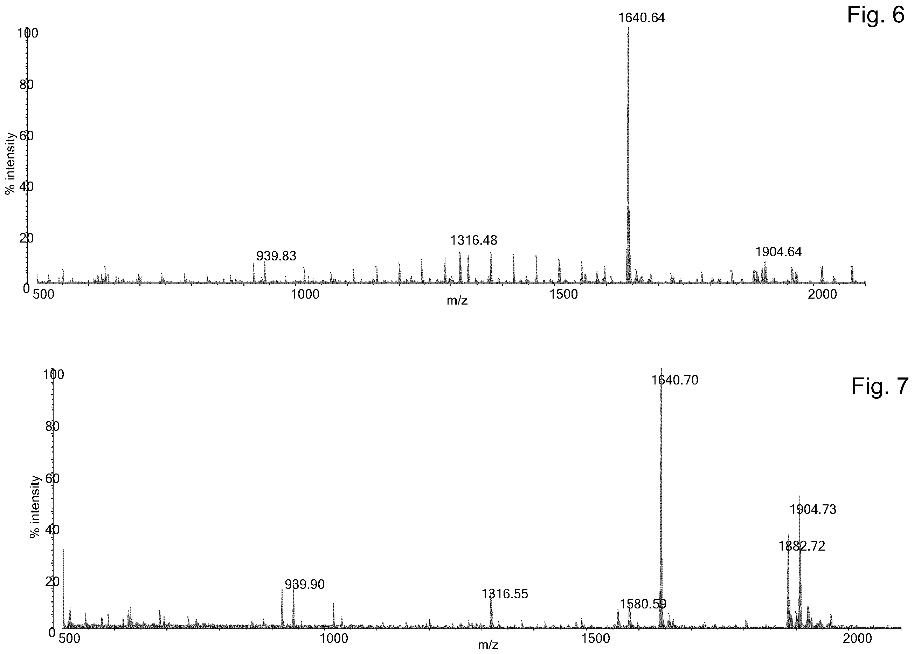

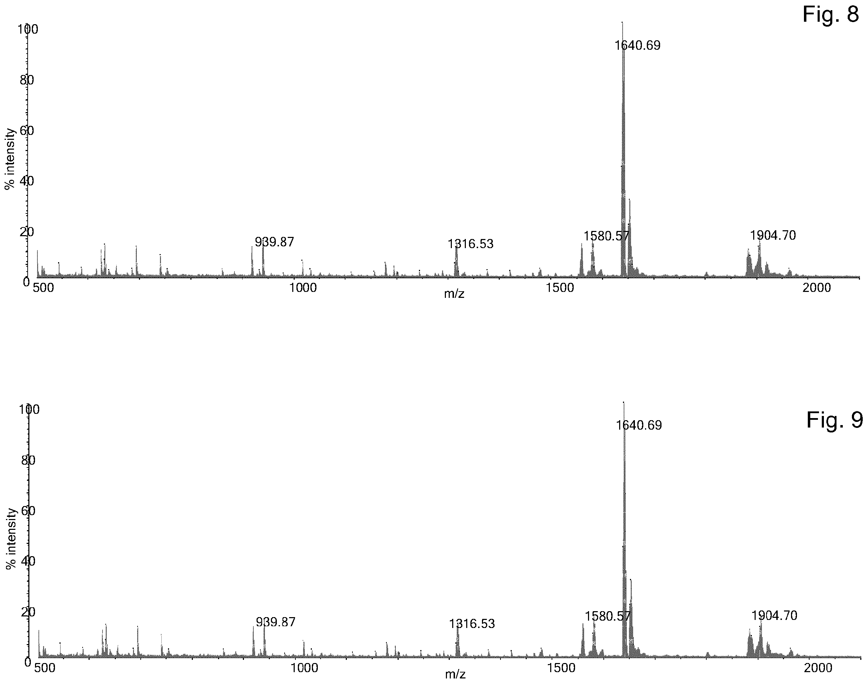

Temperature was set to 68/75/82 °C for a period of 3 generation times for each temperature and afterwards a biomass sample was taken. The relative abundance of the 5 main lipid fractions was determined via MALDI-MS (positive ion mode; without addition of Na$^+$). The results are displayed in Figs. 1 and 5 to 8.

1.2 Influence of growth rate

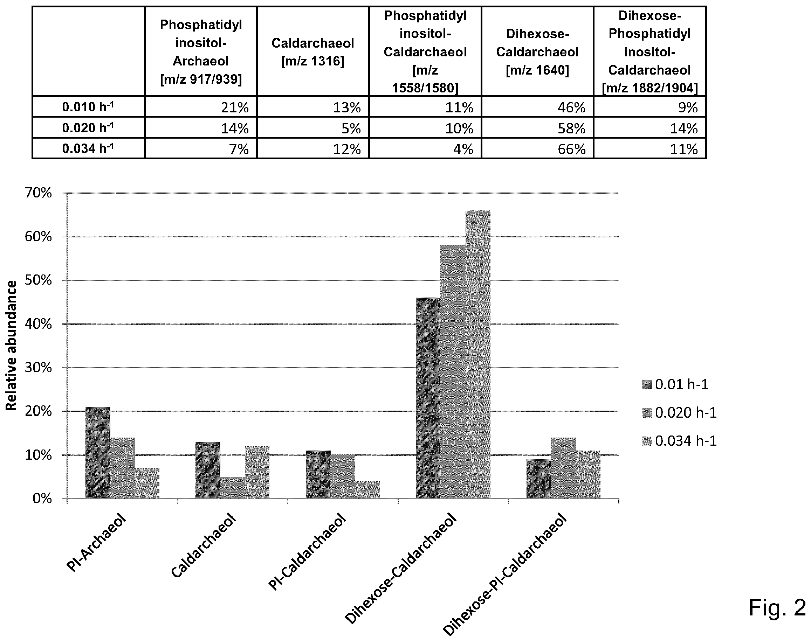

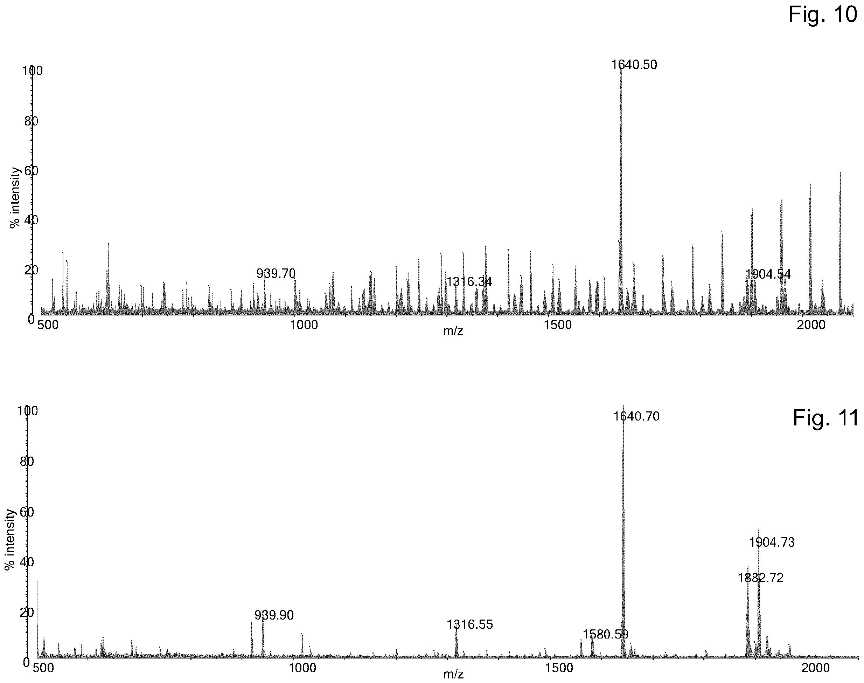

Growth rate was set to 0.010/0.020/0.034 h-1 for a period of 3 generation times for each growth rate and afterwards a biomass sample was taken. The relative abundance of the 5 main lipid fractions was determined via MALDI-MS (positive ion mode; without addition of Na$^+$). The results are displayed in Figs. 2, 9, 10 and 11.

1.3 Influence of process mode

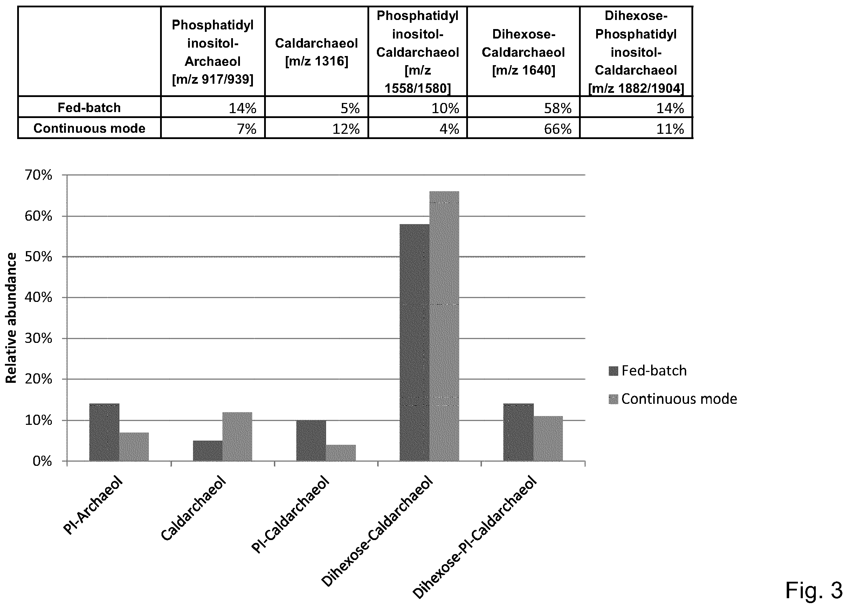

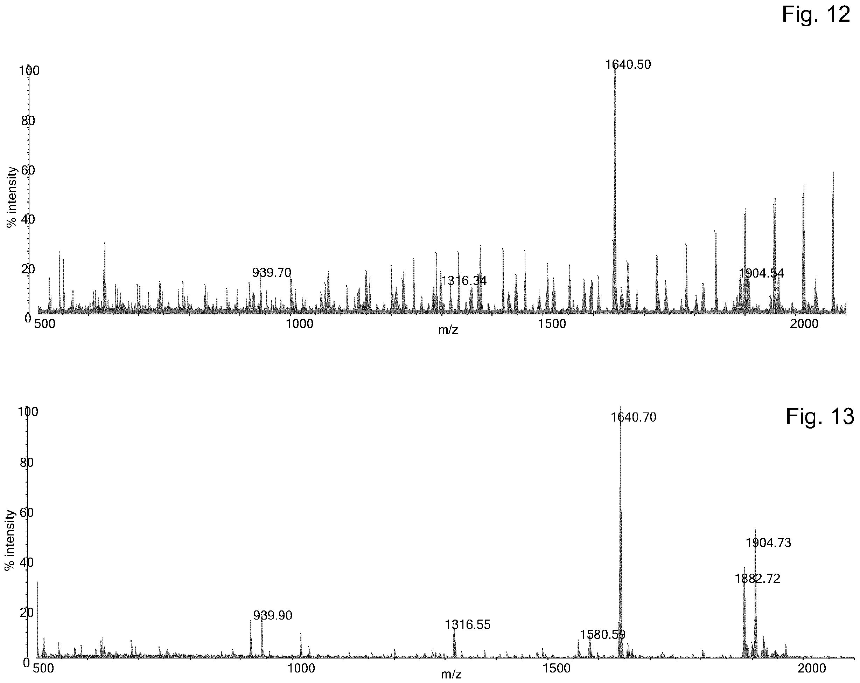

Culture was grown in different process modes (Fed-batch and continuous mode) for a period of 3 generation times for each process mode and afterwards a biomass sample was taken. The relative abundance of the 5 main lipid fractions was determined via MALDI-MS (positive ion mode; without addition of Na$^+$). The results are displayed in Figs. 3, 12 and 13.

2. Materials and Methods

2.1 Upstream: Bioreactor cultivation of *Sulfolobus acidocaldarius DSM639*

For the generation of tailor-made archaeal lipids *S. acidocaldarius* was cultivated in a continuous process mode to ensure constant process conditions over at least 3 generation times. The continuous phase was preceded by a batch and fed-batch phase for buildup of biomass. The volumes in the following paragraphs are exemplarily and can be scaled up or down if required.

The preculture of *Sulfolobus acidocaldarius* DSM 639 was grown in a 100 mL shake flask for 100 h in a rotary oil bath at 75 °C and 100 rpm in a defined medium according to Quehenberger et al. (Journal of Biotechnology (2019) Submission JBIOTEC-D-18-01483; "VD Medium"; see the following table).

Table:

| Composition of the VD Medium. | | | | |
|---|---|---|---|---|
| **Basal salts and trace elements** | | | | |
| $(NH_4)_2SO_4$ | 1.04 | g/L | 7.87 | mM |
| $KH_2PO_4$ | 0.14 | g/L | 1.03 | mM |
| $MgSO_4 \cdot 7 H_2O$ | 0.125 | g/L | 0.51 | mM |
| $CaCl_2 \cdot 2 H_2O$ | 0.035 | g/L | 0.24 | mM |
| $Fe(III)Citrate \cdot H_2O$ | 9.75 | mg/L | 37.08 | $\mu$M |
| $Na_2B_4O_7 \cdot 10 H_2O$ | 0.90 | mg/L | 2.36 | $\mu$M |
| $MnCl_2 \cdot 4 H_2O$ | 0.36 | mg/L | 1.82 | $\mu$M |
| $ZnSO_4 \cdot 7 H_2O$ | 0.066 | mg/L | 0.23 | $\mu$M |
| $CuCl_2 . 2 H_2O$ | 0.010 | mg/L | 0.059 | $\mu$M |
| $Na_2Moo_4 \cdot 2 H_2O$ | 0.006 | mg/L | 0.025 | $\mu$M |
| $CoCl_2 \cdot 6 H_2O$ | 0.002 | mg/L | 0.007 | $\mu$M |
| **C-sources** | | | | |
| Na-L-glutamate | 1.75 | g/L | 10.35 | mM |
| D-glucose | 3.00 | g/L | 16.65 | mM |
| Citric acid | 0.50 | g/L | 2.60 | mM |
| Adjust to pH 3 with $H_2SO_4$ | | | ~130 | mM |

The preculture was transferred aseptically to a culture vessel containing a defined medium according to Quehenberger et al. (2019), yielding a total starting volume for the batch phase of 1.5 L. The Batch phase was performed at 75 °C until a decrease in the $CO_2$ signal was observed and followed by a fed-batch phase at 75 °C (medium containing 10 g/L Na-glutamate and 3 g/L D-glucose, exponential feed, $\mu$ = 0.03 h$^{-1}$) until a total volume of 2.5 L was reached. The fed-batch phase was followed by a continuous cultivation at the desired growth temperature and growth rate. To ensure enough time for the organism to adapt to the specific growth conditions during the continuous phase biomass was harvested after at least 3 generation times.

The calculation of the initial flow rate ($F_0$) of the feed for the exponential phase is based on the mass balance of the carbon source:

$$\dot{V}_{in}c_{i,in} - \dot{V}_{out}c_{i,out} + V_R r_i = V_R \frac{\partial c_i}{\partial t} + c_i \frac{\partial V_R}{\partial t}$$

$\dot{V}$     volumetric flow rate [L/h]
$C_i$     concentration of compound i [g/L]
$V_R$     reactor volume [L]
$r_i$     reaction rate of component i [g/L/h]

Formula 1: General mass balance
After simplification:

$$\dot{V}_{in}c_{i,in} + V_R r_i = V_R \frac{\partial c_i}{\partial t} + c_i \frac{\partial V_R}{\partial t}$$

$V$      volumetric flow rate [L/h]
$C_i$      concentration of compound i [g/L]
$V_R$      reactor volume [L]
$r_i$      reaction rate of component i [g/L/h]

Formula 2: Simplified mass balance for fed-batch

And setting right side (constant terms) to zero and rewriting:

$$F_0 = \frac{\mu \cdot x_0 \cdot V_0}{s_0 \cdot Y_{X/S}}$$

$F_0$      initial feed rate of fed batch [g/h]
$\mu$      specific growth rate [h$^{-1}$]
$x_0$      initial biomass concentration [g/L]
$V_0$      starting volume of reactor [L]
$s_0$      substrate concentration of the feed [g/L]
$Y_{X/S}$      biomass yield [g/g]

Formula 3: Initial feed rate during fed-batch phase (density of broth is assumed as 1 kg/L).

For calculation of the initial Feedrate ($F_0$, Formula 3) $x_0$ was determined via OD600 measurement and a calibration curve for the conversion of OD600 to dry cell weight; $S_0$ was calculated as the sum of the concentrations of Na-glutamate and D-glucose and an empirically determined biomass yield of 0.33 was assumed. $F_0$ was then increased exponentially during the fed-batch phase according to Formula 4.

$$F = F_0 \cdot e^{\mu \cdot t}$$

Formula 4: Calculation of the Feedrate during the fed-batch phase.

Feeding was controlled via the weight of the Feed vessel and a PID-controller was used to compensate for deviations of the feed pump.

During the continuous phase the value for the dilution rate (D) was set equal to the desired growth rate (D=$\mu$), whereby the dilution rate was calculated as the sum of added acid stream and feed. With a known working volume ($V_R$) the feed rate ($F_{conti}$) was calculated via $F_{conti}$=D*$V_R$ (density of broth is assumed as 1 kg/L) .

Dissolved oxygen levels were monitored online and maintained above 15% by aerating with 0.25 to 0.5 vvm (volumes gas per culture volume per minute) pressurized air. Air was substituted with pure oxygen if necessary. pH was monitored online and kept at 3.0 +/- 0.1 by addition of $H_2SO_4$ (9.6% v/v). Feed medium was supplied via a peristaltic pump module following a feed-forward controlled exponential feeding strategy. Mixing was performed at 300 to 500 rpm. $CO_2$ content in the offgas was monitored.

2.2 Downstream: Cell harvest, biomass homogenization and lipid extraction

Cells were harvested via centrifugation at 14,000g for 15 min. After discarding the supernatant, the cell pellet was stored at -20°C for further processing. A cell pellet from 100 mL culture broth per sample (dry cell weight at the time of harvest is shown in Table 1) was thawed and resuspended in 10 mL ice cold ammonium acetate buffer (155 mM). The suspension was homogenized in a glass beaker on an ice/water bath via sonication (6x1 min with 30 sec pause between pulses) . After homogenization the sample was diluted to an OD600 of 1.5 with ice cold ammonium acetate buffer (155 mM) . 3.33 parts of an ice cold 2:1 $CHCl_3$/MeOH mixture was added to 1 part diluted homogenate and the resulting mixture was vortexed for 30 minutes at 1,400 rpm and 4°C. The samples were centrifuged (2 min, 1,000g, 4°C) for phase separation. The organic phase was collected in a glass vial and the sample was evaporated to dryness with $N_2$ at room temperature. The lipids were stored at -20 °C for further analysis.

2.3 Analytics: Identification of lipid fractions with MALDI mass spectrometry

The dried lipid fractions were reconstituted in a 1:3 CHCl$_3$MeOH mixture. These fractions were immediately used for MALDI sample preparation using 2-4-,6-trihydroxy-acetophenon (THAP) as MALDI matrix (roughly 15 mg THAP/mL without or with the addition of sodium chloride for promotion of sodium adduct ions). Finally, matrix and analyte solution were mixed 1:1 for the deposition onto 384-well plate stainless steel MALDI targets: The mass spectrometric instrument used was a Shimadzu MALDI 7090 TOF/RTOF tandem time-of-flight mass spectrometer fitted with a grounded gas collision cell for high-energy CID experiments (E$_{LAB}$ = 20 keV, collision gas: helium). Prior to tandem MS experiments the samples were measured in positive- and negative-ion reflectron TOF-MS. Mass calibration was performed with a commercial peptide mixture supplied by Shimadzu allowing mass calibration up to m/z 3600. In the positive-ion mode, mainly [M+Na]$^+$ and [M+2Na-H]$^+$ adduct ions of the analyte molecules were detected depending on salt doping. Most lipids contained phosphoinositol head groups. All analytes were characterized by high-energy CID TOF/RTOF-MS. In contrast, in negative-ion mode only phosphoinositol-containing lipids were detected (but no neutral lipids) as [M-H]$^-$ ions being also characterized by high-energy CID TOF/RTOF-MS. Up to now, all charged lipid analytes contain a phosphoinositol polar head group. Minor lipids, which may further be present in this mixture, can be analyzed after HPTLC-separation and micropreparative extraction by subsequent MALDI analysis.

## Claims

1. Method for producing a composition comprising archaeal lipids from a *Sulfolobus* cell culture, comprising

   (1) growing a *Sulfolobus* cell culture at a temperature of 75 to 85°C and at a growth rate of 0.025 to 0.035 per hour and extracting the archaeal lipids from the archaeal culture so as to obtain a composition for use for oral retard therapy, or
   (2) growing a *Sulfolobus* cell culture at a temperature of 75 to 85°C and at a growth rate of 0.005 to 0.015 per hour and extracting the archaeal lipids from the archaeal culture so as to obtain a composition for use for oral acute therapy.

2. Method according to claim 1, wherein the *Sulfolobus* cell culture is a culture of *Sulfolobus acidocaldarius* cells.

3. Method according to claim 1 or 2, wherein the composition for use for oral retard therapy comprises a ratio of dihexose-caldarchaeol (2Hex-Cal) to phosphatidyl inositol-archaeol (PI-Arc) of 1:5 to 1:7, especially of 1:5.5 to 1:6.5.

4. Method according to claim 1 or 2, wherein the composition for use for oral acute therapy comprises a ratio of dihexose-caldarchaeol (2Hex-Cal) to phosphatidyl inositol-archaeol (PI-Arc) of 1:2 to 1:4, especially of 1:2.5 to 1:3.5.

5. Method according to any one of claims 1 to 4, wherein the cells are homogenised after culturing, preferably via homogenization, especially high-pressure homogenization, or sonication.

6. Method according to claim 5, wherein the homogenised cells are mixed with a buffer, preferably with ammonium acetate buffer, especially with an ammonium acetate buffer having 100 to 200 mM ammonium acetate.

7. Method according to any one of claims 1 to 6, wherein the archaeal lipids are obtained by extraction from the aqueous phase comprising the cells and/or the homogenised cells into an organic phase, preferably by extraction into a chloroform/methanol organic phase, and phase separation of the organic phase from the aqueous phase.

8. Method according to claim 7, wherein the archaeal lipids are obtained by evaporation of the organic phase, preferably by evaporation of the organic phase to dryness.

9. Method according to any one of claims 1 to 8, wherein the archaeal lipids obtained are combined with a pharmaceutically active drug, preferably in combination with a pharmaceutically acceptable carrier, so as to obtain a composition comprising archaeal lipids and the pharmaceutically active drug.

10. Method according to any one of claims 1 to 9, wherein the archaeal lipids are obtained by supercritical fluid extraction, especially extraction with supercritical CO$_2$.

11. Composition comprising archaeal lipids obtainable by a method according to any one of claims 1 to 10.

12. Composition comprising archaeal lipids, preferably according to claim 11, wherein the composition comprises a ratio of dihexose-caldarchaeol (2Hex-Cal) to phosphatidyl inositol-archaeol (PI-Arc) of 1:5 to 1:7 or of 1:2 to 1:4.

13. Composition according to claim 11 or 12, further comprising a drug, preferably a drug for human medical use, especially a drug which is subject to an authorization to place the product on the market as a medicinal product issued in the EU or in the US.

14. Composition according to any one of claims 11 to 13, wherein the archaeosomes comprise at least 5 % w/w, preferably at least 10 % w/w, PI-Arc and at least 50 % w/w 2Hex-Cal (% w/w as % w/w of total lipid).

**Patentansprüche**

1. Verfahren zur Herstellung einer Zusammensetzung, die Archaea-Lipide aus einer *Sulfolobus*-Zellkultur aufweist, aufweisend

   (1) Züchten einer *Sulfolobus*-Zellkultur bei einer Temperatur von 75 bis 85 °C und bei einer Wachstumsrate von 0,025 bis 0,035 pro Stunde und Extrahieren der Archaea-Lipide aus der Archaea-Kultur, um eine Zusammensetzung zur Anwendung für eine orale Retard-Therapie zu erhalten, oder
   (2) Züchten einer Sulfolobus-Zellkultur bei einer Temperatur von 75 bis 85 °C und bei einer Wachstumsrate von 0,005 bis 0,015 pro Stunde und Extrahieren der Archaea-Lipide aus der Archaea-Kultur, um eine Zusammensetzung zur Anwendung für eine orale Akuttherapie zu erhalten.

2. Verfahren nach Anspruch 1, wobei die *Sulfolobus*-Zellkultur eine Kultur aus *Sulfolobus acidocaldarius-Zellen* ist.

3. Verfahren nach Anspruch 1 oder 2, wobei die Zusammensetzung zur Anwendung für die orale Retard-Therapie ein Verhältnis von Dihexose-Caldarchaeol (2Hex-Cal) zu Phosphatidylinositol-Archaeol (PI-Arc) von 1:5 bis 1:7, insbesondere von 1:5,5 bis 1:6,5 aufweist.

4. Verfahren nach Anspruch 1 oder 2, wobei die Zusammensetzung zur Anwendung für die orale Akuttherapie ein Verhältnis von Dihexose-Caldarchaeol (2Hex-Cal) zu Phosphatidylinositol-Archaeol (PI-Arc) von 1:2 bis 1:4, insbesondere von 1:2,5 bis 1:3,5 aufweist.

5. Verfahren nach einem der Ansprüche 1 bis 4, wobei die Zellen nach dem Kultivieren, vorzugsweise mittels Homogenisierung, insbesondere Hochdruckhomogenisierung, oder Beschallung, homogenisiert werden.

6. Verfahren nach Anspruch 5, wobei die homogenisierten Zellen mit einem Puffer, vorzugsweise mit Ammoniumacetatpuffer, insbesondere mit einem Ammoniumacetatpuffer mit 100 bis 200 mM Ammoniumacetat, gemischt werden.

7. Verfahren nach einem der Ansprüche 1 bis 6, wobei die Archaea-Lipide durch Extraktion aus der wässrigen Phase, welche die Zellen und/oder die homogenisierten Zellen aufweist, in eine organische Phase, vorzugsweise durch Extraktion in eine organische Chloroform/Methanol-Phase, und Phasentrennung der organischen Phase von der wässrigen Phase erhalten werden.

8. Verfahren nach Anspruch 7, wobei die Archaea-Lipide durch Verdampfung der organischen Phase, vorzugsweise durch Verdampfung der organischen Phase bis zur Trocknung, erhalten werden.

9. Verfahren nach einem der Ansprüche 1 bis 8, wobei die erhaltenen Archaea-Lipide mit einem pharmazeutisch aktiven Arzneimittel, vorzugsweise in Kombination mit einem pharmazeutisch verträglichen Träger, kombiniert werden, um eine Zusammensetzung zu erhalten, die Archaea-Lipide und das pharmazeutisch wirksame Arzneimittel aufweist.

10. Verfahren nach einem der Ansprüche 1 bis 9, wobei die Archaea-Lipide durch überkritische Fluid-Extraktion, insbesondere Extraktion mit überkritischem $CO_2$, erhalten werden.

11. Zusammensetzung, die Archaea-Lipide aufweist, die durch ein Verfahren nach einem der Ansprüche 1 bis 10 erhalten werden kann.

**12.** Zusammensetzung, die Archaea-Lipide aufweist, vorzugsweise nach Anspruch 11, wobei die Zusammensetzung ein Verhältnis von Dihexose-Caldarchaeol (2Hex-Cal) zu Phosphatidylinositol-Archaeol (PI-Arc) von 1:5 bis 1:7 von 1:2 bis 1:4 aufweist.

**13.** Zusammensetzung nach Anspruch 11 oder 12, die ferner ein Arzneimittel aufweist, vorzugsweise ein Arzneimittel für die medizinische Anwendung beim Menschen, insbesondere ein Arzneimittel, das einer Genehmigung zum Inverkehrbringen des Produkts als Arzneimittel unterliegt, die in der EU oder in den USA erteilt wurde.

**14.** Zusammensetzung nach einem der Ansprüche 11 bis 13, wobei die Archaeosomen mindestens 5 Gew.-%, vorzugsweise mindestens 10 Gew.-%, PI-Arc und mindestens 50 Gew.-% 2Hex-Cal (Gew.-% als Gew.-% des Gesamtlipids) aufweisen.

## Revendications

**1.** Procédé pour produire une composition comprenant des lipides archéens provenant d'une culture cellulaire de *Sulfolobus,* comprenant

(1) la croissance d'une culture cellulaire de *Sulfolobus* à une température de 75 à 85°C et à une vitesse de croissance de 0,025 à 0,035 par heure et l'extraction des lipides archéens de la culture archéenne de manière à obtenir une composition destinée pour utilisation dans une thérapie retard orale, ou
(2) la croissance d'une culture cellulaire de *Sulfolobus* à une température de 75 à 85°C et à une vitesse de croissance de 0,005 à 0,015 par heure et l'extraction des lipides archéens de la culture archéenne de manière à obtenir une composition pour utilisation dans une thérapie orale aiguë.

**2.** Procédé selon la revendication 1, dans lequel la culture cellulaire de *Sulfolobus* est une culture de cellules de *Sulfolobus acidocaldarius.*

**3.** Procédé selon la revendication 1 ou 2, dans lequel la composition pour utilisation dans une thérapie retard orale comprend un rapport de dihexose-caldarchaeol (2Hex-Cal) à phosphatidyl inositol-archaeol (PI-Arc) de 1:5 à 1:7, en particulier de 1:5,5 à 1:6,5.

**4.** Procédé selon la revendication 1 ou 2, dans lequel la composition pour utilisation dans une thérapie orale aiguë comprend un rapport de dihexose-caldarchaeol (2Hex-Cal) à phosphatidyl inositol-archaeol (PI-Arc) de 1:2 à 1:4, en particulier de 1:2,5 à 1:3,5.

**5.** Procédé selon l'une quelconque des revendications 1 à 4, dans lequel les cellules sont homogénéisées après culture, de préférence via homogénéisation, en particulier homogénéisation à haute pression, ou sonication.

**6.** Procédé selon la revendication 5, dans lequel les cellules homogénéisées sont mélangés avec un tampon, de préférence avec un tampon acétate d'ammonium, en particulier avec un tampon acétate d'ammonium ayant de l'acétate d'ammonium 100 à 200 mM.

**7.** Procédé selon l'une quelconque des revendications 1 à 6, dans lequel les lipides archéens sont obtenus par extraction à partir de la phase aqueuse comprenant les cellules et/ou les cellules homogénéisées dans une phase organique, de préférence par extraction dans une phase organique chloroforme/méthanol, et séparation de phases de la phase organique d'avec la phase aqueuse.

**8.** Procédé selon la revendication 7, dans lequel les lipides archéens sont obtenus par évaporation de la phase organique, de préférence par évaporation de la phase organique à siccité.

**9.** Procédé selon l'une quelconque des revendications 1 à 8, dans lequel les lipides archéens obtenus sont combinés avec un médicament pharmaceutiquement actif, de préférence en combinaison avec un vecteur pharmaceutiquement acceptable, de manière à obtenir une composition comprenant des lipides archéens et le médicament pharmaceutiquement actif.

**10.** Procédé selon l'une quelconque des revendications 1 à 9, dans lequel les lipides archéens sont obtenus par extraction par fluide supercritique, en particulier extraction avec COz supercritique.

**11.** Composition comprenant des lipides archéens pouvant être obtenue par un procédé selon l'une quelconque des revendications 1 à 10.

**12.** Composition comprenant des lipides archéens, de préférence selon la revendication 11, dans laquelle la composition comprend un rapport de dihexose-caldarchaeol (2Hex-Cal) à phosphatidyl inositol-archaeol (PI-Arc) de 1:5 à 1:7 ou de 1:2 à 1:4.

**13.** Composition selon la revendication 11 ou 12, comprenant en outre un médicament, de préférence un médicament à usage médical humain, en particulier un médicament qui fait l'objet d'une autorisation de mise sur le marché comme produit médicinal délivrée dans l'UE ou aux USA.

**14.** Composition selon l'une quelconque des revendications 11 à 13, dans laquelle les archéosomes comprennent au moins 5 % p/p, de préférence au moins 10 % p/p, de PI-Arc et au moins 50 % p/p de 2Hex-Cal (% p/p en % p/p de lipides totaux).

EP 3 937 901 B1

| | Phosphatidyl inositol-Archaeol [m/z 917/939] | Caldarchaeol [m/z 1316] | Phosphatidyl inositol-Caldarchaeol [m/z 1558/1580] | Dihexose-Caldarchaeol [m/z 1640] | Dihexose-Phosphatidyl inositol-Caldarchaeol [m/z 1882/1904] |
|---|---|---|---|---|---|
| | | | | | |
| 68 °C | 17% | 9% | 9% | 52% | 13% |
| 75 °C | 7% | 12% | 4% | 66% | 11% |
| 82 °C | 21% | 13% | 11% | 46% | 9% |

Fig. 1

| | Phosphatidyl inositol-Archaeol [m/z 917/939] | Caldarchaeol [m/z 1316] | Phosphatidyl inositol-Caldarchaeol [m/z 1558/1580] | Dihexose-Caldarchaeol [m/z 1640] | Dihexose-Phosphatidyl inositol-Caldarchaeol [m/z 1882/1904] |
|---|---|---|---|---|---|
| 0.010 h$^{-1}$ | 21% | 13% | 11% | 46% | 9% |
| 0.020 h$^{-1}$ | 14% | 5% | 10% | 58% | 14% |
| 0.034 h$^{-1}$ | 7% | 12% | 4% | 66% | 11% |

Fig. 2

| | Phosphatidyl inositol-Archaeol [m/z 917/939] | Caldarchaeol [m/z 1316] | Phosphatidyl inositol-Caldarchaeol [m/z 1558/1580] | Dihexose-Caldarchaeol [m/z 1640] | Dihexose-Phosphatidyl inositol-Caldarchaeol [m/z 1882/1904] |
|---|---|---|---|---|---|
| Fed-batch | 14% | 5% | 10% | 58% | 14% |
| Continuous mode | 7% | 12% | 4% | 66% | 11% |

Fig. 3

| Phosphatidyl inositol-Archaeol [m/z 917/939] | |
| Caldarchaeol [m/z 1316] | |
| Phosphatidyl inositol-Caldarchaeol [m/z 1558/1580] | |
| Dihexose-Caldarchaeol [m/z 1640] | |
| Dihexose-Phosphatidyl inositol-Caldarchaeol [m/z 1882/1904] | |

Fig. 4

Fig. 5

Fig. 6

Fig. 7

Fig. 8

Fig. 9

Fig. 10

Fig. 11

Fig. 12

Fig. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5098588 A
- WO 2007112567 A1
- DE 102012216378 A1
- US 20170152533 A1
- US 6316260 B1
- EP 1999137 B1
- EP 0883624 B1
- EP 2109459 B1

### Non-patent literature cited in the description

- **VAN HOOGEVEST.** *Eur. J. Pharm. Sci.,* 2017, vol. 108, 1-12
- **SZOKA et al.** *Ann. Rev. Biophys. Bioeng.,* 1980, vol. 9, 467-508
- **KAUR et al.** *Drug delivery,* 2016, vol. 23, 2497
- **PATEL et al.** *Critical Reviews in Biotechnology,* 1999, vol. 19, 317
- **JENSEN et al.** *Life,* 2015, vol. 5, 1539
- **SPROTT et al.** *J. Bact.,* 1991, vol. 173, 3907
- **SILIAKUS et al.** *Extremophiles,* 2017, vol. 21, 651-670
- **JAIN et al.** *Frontiers in Microbiol.,* 2014, vol. 5
- **SPROTT et al.** *Can. J. Microbiol.,* 1997, vol. 43, 467-476
- **ANDRADE.** *J. Clin. Psychiatry,* 2015, vol. 76, e995-e999
- Controlling drug delivery. **PERRIE et al.** Pharmaceutics: drug Delivery and Targeting. Pharmaceutical Press, 2012
- **BROCK et al.** *Arch. Mikrobiol.,* 1972, vol. 84, 54-68
- **HANIF et al.** *Int. J. Mol. Sci.,* 2012, vol. 13, 3022-3037